# EUROPEAN PATENT APPLICATION

(11) **EP 0 569 098 A2**
(43) Date of publication of application: **10.11.1993**
(21) Application number: 93201286.7
(22) Date of filing: 06.05.1993
(51) Int. Cl.: C07D 315/00, C07C 45/41, C07C 51/235, B01J 23/34, B01J 23/84, B01J 37/03

(54) **Conversion of cycloalkanone into lactone**

(30) Priority: 07.05.1992 NL 9200817
(71) Applicant: DSM N.V., NL-6411 TE Heerlen (NL)
(72) Inventor: van Geem, Paul Christiaan, NL-6174 RC Schinnen (NL)

(57) **Abstract**

The invention relates to a process for the oxidation of a cycloalkanone containing 6-12 carbon atoms to the corresponding lactone in the presence of a benzaldehyde with the aid of an oxygen-containing gas, the benzoic acid that is formed in the oxidation being hydrogenated to benzaldehyde and the benzaldehyde thus obtained being used again in the oxidation of cycloalkanone.

A catalyst that is very suitable for the conversion of a benzoic acid into the corresponding benzaldehyde can be obtained via coprecipitation of a manganese salt, a salt from which an acid support is formed at a pH between 7 and 10 in the presence of a zinc salt and optionally a copper salt, through calcination, after precipitation, at a temperature of between 300 and 700°C and then reduction with the aid of a hydrogen-containing gas mixture.

## Description

The invention relates to a process for the oxidation of a cycloalkanone containing 6-12 carbon atoms to the corresponding lactone in the presence of a benzaldehyde with the aid of an oxygen-containing gas.

Since the mid sixties it has been known to convert a cycloalkanone such as cyclohexanone into caprolactone by means of a Baeyer-Villiger oxidation (e.g. from NL-A-6501332). In this Baeyer-Villiger oxidation an aldehyde such as benzaldehyde is used as an auxiliary. The aldehyde reacts with oxygen to form a peracid which, in turn, effects the desired oxidation to caprolactone. The conversion of cyclohexanone by means of the Baeyer-Villiger oxidation to caprolactone, which can in turn be converted into caprolactam with the aid of ammonia, presents the great advantage that no ammonium sulphate is formed in the preparation of caprolactam via caprolactone. Benzoic acid is formed though.

According to US-A-3025306 it is an advantage that two useful products (i.g. caprolactone and benzoic acid) are obtained with a Baeyer-Villiger oxidation. The benzoic acid can be worked up as such but it is very difficult to obtain the product in such a pure form that it can be sold as, for example, a preservative. This purification also entails very high costs.

The aim of the invention now is to provide an economically attractive route to lactone and lactam in which no ammonium sulphate is formed as a byproduct and in which benzoic acid does not have to be sold as a byproduct either.

This aim, now, is achieved because benzoic that is formed in the oxidation reaction is hydrogenated to benzaldehyde and because the benzaldehyde thus obtained is used again in the oxidation of cycloalkanone.

NL-A-6501332 teaches that the benzoic acid formed after conversion of cyclohexanone into caprolactone can be converted into phenol (step 1), which can then be converted into cyclohexanone (step 2) which can be used as starting material for the caprolactam production. This is disadvantageous because several process steps are required. Moreover the selectivity of cyclohexanone obtained from benzoic acid via phenol according to NS-A-6501332 is not high. An advantage of the present invention is that the benzoic acid can be converted to benzaldehyde in one process step. Reduction to one process step will result in an considerable reduction of handware compared to NL-A-6501332. Another disadvantage of NL-A-6501332 is that the main starting material is benzaldehyde, which is not easily obtainable. By converting the benzoic acid to benzaldehyde the main starting material will be cyclohexanone which is more easily obtainable than benzaldehyde. Because the selectivity of benzaldehyde from benzoic acid is higher than the above mentioned selectivity of cyclohexanone from benzoic acid, less material is lost and less benzaldehyde has to be suppleted to achieve the 1:1 ratio of cyclohexanone to benzaldehyde. Because of the above the process can be carried out in a cost effective manner.

In the process according to the invention unsubstituted benzaldehyde, but also benzaldehyde that is mono- or di-substituted with halogen or with alkyl groups containing 1-4 carbon atoms, may be used as benzaldehyde. Examples of suitable benzaldehyde compounds are o-Cl-benzaldehyde, m-Cl-benzaldehyde, p-Cl-benzaldehyde, 2,6-dimethylbenzaldehyde and p-t.butyl-benzaldehyde. In the application 'benzaldehyde' will be understood to include substituted benzaldehyde.

As cycloalkanone containing between 6 and 12 carbon atoms use is preferably made of a cycloalkanone containing 6, 8 or 12 carbon atoms. Suitable cycloalkanones are cyclohexanone, cyclooctanone and cyclododecanone. Cyclohexanone and cyclooctanone, with which ε-caprolactone and capryllactone can be prepared, are particularly suitable.

The lactone can be prepared through oxidation of cycloalkanone in the liquid phase with the aid of gas containing molecular oxygen in the presence of a benzaldehyde, under the influence of a catalyst, in which a corresponding benzoic acid is also formed. The equation of the reaction of cyclohexanone to ε-caprolactone with the aid of unsubstituted benzaldehyde is given as an example. 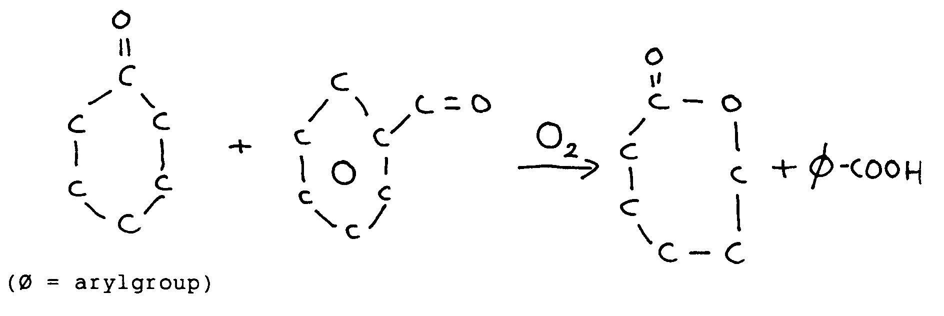
The gas containing molecular oxygen, for example air, can be passed through the reaction mixture in a simple manner. The reaction is carried out at a temperature below 100°C, preferably between 20 and 50°C. The reaction takes place at atmospheric pressure, but elevated pressures of i.g. 5, 10, 25, and 50 atm. are also possible.

As catalysts use can be made of compounds of metals of group VIII of the Periodic Table; see Chemical Letters 1991, pp. 641-644 of 'the Chemical Society of Japan'.

Suitable solvents in which the reaction can take place are hydrocarbons, chlorinated hydrocarbons, for example chlorobenzene, or esters, for example ethylacetate.

The reaction product of the oxidation can be worked up in the usual manner, for example through distillation or extraction.

The crude benzoic acid thus obtained is preferably distilled first before being fed to a hydrogenation reactor. This distillation is carried out using for example a distillation unit with a partial condenser.

The hydrogenation of benzoic acid to benzaldehyde can be carried out in a manner known per se, under the influence of a hydrogenation catalyst and a gas mixture containing hydrogen.

The hydrogenation of the benzoic acid preferably takes place in the gas phase at a temperature between 250°C and 600°C. More in particular the hydrogenation takes place in the presence of a catalyst containing manganese on an acid support as described in NL-A-8701063. This catalyst is prepared via coprecipitation of a manganese salt and a salt from which an acid support is formed at a pH between 7 and 10, followed by calcination at a temperature between 300 and 700°C, and, in a last step, reduction using a hydrogen-containing gas mixture. A hydrogenation according to NL-A-8701063 enables the production of a very high yield of benzaldehyde in the gas phase.
The catalyst used is an oxide of manganese on an acid support in the form of an oxide.

As salt from which an acid support is formed use can be made of a salt from which on coprecipitation for example oxides of aluminium, zirconium, titanium, cerium, hafnium and/or niobium are formed. Preferably a nitrate of such an element is used. A suitable coprecipitator is for example NH₄OH or K₂CO₃.

The catalysts usually contain 5-50 wt.% manganese, calculated as metal. The catalyst preferably contains 10-35 wt.% manganese.

In this case the hydrogenation preferably takes place at a temperature of 400-500°C.

From an energy-saving and hence also cost-saving viewpoint it is desirable to carry out the hydrogenation at lower temperatures.

Zinc appears to be a suitable promoter to effect this. The presence of copper moreover enhances the effects of zinc. The selectivity and activity with respect to the benzaldehyde increase at lower reaction temperatures, which is important in connection with the prevention of the undesired formation of byproducts such as toluene, benzene and benzyl alcohol. If zinc and/or copper are used as promoters the hydrogenation reaction is preferably carried out at a temperature between 300 and 400°C.

The process for the preparation of the catalyst using zinc and optionally copper does not differ substantially from the process without these promoters. The soluble salts are added to a solution of manganese salt, after which metal hydroxides are coprecipitated.

Suitable zinc compounds are in particular soluble zinc salts such as zinc nitrate, zinc sulphate, zinc chloride, zinc acetate.

Suitable copper compounds are in particular soluble copper salts such as copper nitrate, copper sulphate, copper chloride.

Such manganese oxide catalysts modified with promoters are suitable for the preparation of all kinds of benzaldehydes through hydrogenation of a benzoic acid. Benzoic acid may for example be substituted in one or more places in the aromatic ring with an alkyl group containing 1-6 C atoms, an alkoxy group containing 1-6 C atoms, a hydroxyl group and a halogen atom. Other substituents are also possible. The substituents may be at the ortho, meta and para positions.

After the hydrogenation the gas mixture is cooled and excess hydrogen is recirculated. The water and benzene formed in the hydrogenation are removed in an azeotropic drying column, after which benzaldehyde is distilled.

The invention is explained with reference to the following, non-limiting, examples.

### Examples

### Preparation of the catalyst

Catalysts were prepared by dissolving Mn(NO₃)₂.4H₂O, Al(NO₃)₃.9H₂O, Cu(NO₃)₂.5H₂O and Zn(NO₃)₂.6H₂O in water to obtain a 1 M solution (relative to nitrate). Then a precipitate was formed by adding a 1 M solution of K₂CO₃ or NH₄OH to the solution under the conditions indicated in Table 1.

After filtration and drying the catalyst was reduced and screened to obtain 1-3-mm large particles. Then the catalyst was calcined at 200°C for 2 hours, followed by calcination at 500°C for 3 hours.

The compositions of these catalysts are as indicated in Table 1; Table 2 shows the results of a metal element analysis.

**TABLE 1**

| No. | catalyst preparation | | | composition |
|---|---|---|---|---|
| | solution pH | temp. | base | |
| 1 | 7.0 | 45 | K₂CO₃ | MnO₂/0.1 CuO 1.1 ZnO.ZnAl₂O₄ |
| 2 | 7.0 | 45 | K₂CO₃ | MnO₂/ZnAl₂O₄ |
| 3 | 7.0 | 45 | K₂CO₃ | MnO₂/ZnO ZnAl₂O₄ |
| 4 | 5.0 | 80 | K₂CO₃ | MnO₂/ZnO ZnAl₂O₄ |
| 5 | 7.0 | 45 | NH₄OH | MnO₂/ZnO ZnAl₂O₄ |
| 6 | 7.0 | 45 | NH₄OH | MnO₂/Al₂O₄ |

**TABLE 2**

| Cat. No. | Mn | Zn | Al | Cu |
|---|---|---|---|---|
| 1 | 10.6 | 27.0 | 22.5 | 1.2 |
| 2 | 11.2 | 26.6 | 23.1 | - |
| 3 | 11.2 | 36.0 | 15.8 | - |
| 4 | 3.5 | 42.5 | 19.0 | - |
| 5 | 13.0 | 31.0 | 19.8 | - |
| 6 | 10.1 | - | 36.1 | - |

### Examples I-XI

The benzoic acid was introduced into a saturator that was maintained at a temperature of 150°C. Hydrogen and nitrogen were added to the saturator containing benzoic acid.
The reactor with a diameter of 8 mm was provided with 5 ml of catalyst having a particle size of between 0.5 and 0.8 mm. The reactor was heated to a temperature of between 300 and 450°C with the aid of a tube furnace. The feed rate of the hydrogen was 35 ml/h and that of the nitrogen was 4.75 ml/h. The actual contact time of the reaction mixture was 0.2 seconds at 350°C. After the reactor the product stream (to which a DMF flow of 6.9 g/h had been added after the reaction) was collected in two coolers of 15 and -3°C for 30 minutes. Then the components were analysed.

Table 3 shows the temperature at which the reaction was carried out and the results that were obtained with catalysts 1-6.

**TABLE 3**

| Ex. | Cat. | Temp. (°C) | selectivity towards benzaldehyde | degree of conversion of benzoic acid |
|---|---|---|---|---|
| I | 1 | 330 | 88.3 | 98.9 |
| II | 2 | 350 | 95.2 | 85.7 |
| III | 2 | 380 | 78.2 | 100 |
| IV | 3 | 350 | 91.0 | 48.4 |
| V | 3 | 380 | 83.8 | 100 |
| VI | 4 | 350 | 95.0 | 53.2 |
| VII | 4 | 380 | 74.3 | 100 |
| VIII | 5 | 350 | 96.4 | 70.0 |
| IX | 5 | 380 | 79.7 | 100 |
| X | 6 | 414 | 84 | 89 |
| XI | 6 | 435 | 81 | 100 |

### Example XII

166.5 g (1.70 mol) of cyclohexanone and 1.5 mg of cobalt naphthenate were brought to a temperature of 40°C in a reaction vessel equipped with a feed device, a reflux cooler, a stirrer, a thermometer and a gas distribution device. Next a mixture of 114.5 g (1.17 mol) of cyclohexanone, 304 g (2.87 mol) of benzaldehyde and 4.5 mg of cobalt naphthenate were slowly added, in 4 hours, with stirring, while oxygen was passed through (200 l/h). The temperature was maintained at 40-45°C. Then oxygen was passed through for another 30 minutes, during which the stirring was continued.

Next the reaction mixture was filtered (191 g of benzoic acid) and the filtrate was extracted with the aid of ether after the addition of 1.2 l of water in which 200 g of sodium carbonate was dissolved. The ether extract was distilled, 0.96 mol cyclohexanone, 0.30 mol benzaldehyde and 1.55 ε-caprolactone being separated and 0.11 mol polymeric lactone remaining as a residue.

By adding sulphuric acid benzoic acid was caused to precipitate from the aqueous phase, which was separated through filtration (107 g) so that a total of 298 g (2.44 mol) of benzoic acid was obtained. 10.1 g of adipic acid was separated from the filtrate via extraction using butanol.

In this manner 1.91 mol (66.5%) cyclohexanone and 2.57 mol (89.5%) benzaldehyde were converted. The total yield of lactone and polymeric lactone is 87% and the yield of benzoic acid is 95%.

The amount of 298 g of benzoic acid obtained was converted into benzaldehyde together with 50 g of industrial benzoic acid, in a manner described in Example I. After purification 300 g of benzaldehyde was available, which was used for a next oxidation of cyclohexanone to caprolactone.

## Claims

1. Process for the oxidation of a cycloalkanone containing 6-12 carbon atoms to the corresponding lactone in the presence of a benzaldehyde, with the aid of an oxygen-containing gas, characterised in that benzoic acid that is formed in the oxidation is hydrogenated to benzaldehyde and that benzaldehyde thus obtained is used again in the oxidation of cycloalkanone.

2. Process according to claim 1, characterised in that the hydrogenation of benzoic acid to benzaldehyde takes place in the gas phase in the presence of a manganese-containing catalyst on an acid support, obtainable through coprecipitation of a manganese salt and a salt from which an acid support is formed at a pH between 7 and 10, followed by calcination at a temperature of between 300 and 700°C and, in a last step, reduction with the aid of a hydrogen-containing gas mixture.

3. Process according to claim 2, characterised in that zinc, and optionally copper, is present as promoter in the catalyst.

4. Process according to any one of claims 1-3, characterised in that cyclohexanone or cyclooctanone is used as cycloalkanone.

5. Process according to any one of claims 1-4, characterised in that unsubstituted benzaldehyde or benzaldehyde that is mono- or disubstituted with halogen or alkyl containing 1-4 carbon atoms is used as benzaldehyde.

6. Catalyst suitable for the conversion of a benzoic acid into the corresponding benzaldehyde obtainable through coprecipitation of a manganese salt, a salt from which an acid support is formed at a pH between 7 and 10 in the presence of a zinc salt and optionally a copper salt, followed by precipitation and calcination at a temperature of between 300°C and 700°C and then reduction with the aid of a hydrogen-containing gas mixture.

7. Process for the hydrogenation of benzoic acid to benzaldehyde by hydrogenating unsubstituted benzoic acid or a benzoic acid that is substituted in one or several places in the aromatic ring with an alkyl group containing 1-6 C atoms, an alkoxy group containing 1-6 C atoms, a hydroxyl group or a halogen atom, in the gas phase, with the aid of a catalyst according to claim 6.

8. Application of a catalyst according to claim 6 for the hydrogenation of benzoic acid or derivatives thereof.
